# FASCICULE DE BREVET EUROPEEN

(11) **EP 1 117 408 B1**
(45) Date de publication et mention de la délivrance du brevet: **16.06.2004**
(21) Numéro de dépôt: 99946279.9
(22) Date de dépôt: 01.10.1999
(51) Int. Cl.: A61K 31/554, A61P 27/02

(54) **UTILISATION DU DILTIAZEM POUR LE TRAITEMENT DE PATHOLOGIES DE LA RETINE**
VERWENDUNG VON DILTIAZEM ZUR BEHANDLUNG VON PATHOLOGIEN DER NETZHAUT
USE OF DILTIAZEM FOR TREATING RETINAL PATHOLOGIES

(30) Priorité: 02.10.1998 FR 9812364
(43) Date de publication de la demande: 25.07.2001
(73) Titulaire: INSTITUT NATIONAL DE LA SANTE ET DE LA RECHERCHE MEDICALE (INSERM), 75654 Paris Cédex 13 (FR); UNIVERSITE LOUIS PASTEUR, 67000 Strasbourg (FR)
(72) Inventeur: PICAUD, Serge, F-67000 Strasbourg (FR); FRASSON, Maria, F-68250 Rouffach (FR); SAHEL, José, F-67000 Strasbourg (FR); DREYFUS, Henri, F-67000 Strasbourg (FR)
(74) Mandataire: Grosset-Fournier, Chantal Catherine
(86) Numéro de dépôt international: PCT/FR1999/002346
(87) Numéro de publication internationale: WO 2000/020006

(56) Documents cités:
- WO-A-90/06123
- WO-A-96/00073
- WO-A-96/03985
- WO-A-98/50065
- D.P. EDWARD ET AL.: "Amelioration of Light-Induced Retinal Degeneration by a Calcium Overload Blocker" ARCHIVES OF OPHTHALMOLOGY, vol. 109, no. 4, 1991, pages 554-562, XP002105534
- D.P. EDWARD ET AL.: "The Amelioration of Light Induced Retinal Degeneration by Flunarizine, a Calcium Channel Blocker" OPHTAHLMOL. VISUAL SCI., vol. 31, no. 4, 1990, page 293 XP002105535
- I. SAHLY ET AL.: "Calcium channel blockers inhibit retinal degenration in the retinal-degeneration-B mutant of drosophila" PROCEEDINGS OF THE NATIONAL ACADEMY OF SCIENCES USA, vol. 89, no. 1, 1992, page 435-9 XP002105536
- M.O.M. TSO: "In Search of Pharmacotherapy for Photoreceptor Degeneration" FOLIA OPHTHALMOLOGICA JAPONICA, vol. 43, no. 2, 1992, pages 139-140, XP002105537
- C.J. MEDRANO ET AL.: "Oxygen Consumption in the Rat Outer and Inner Retina: Light- and Pharmacologically-Induced Inhibition" EXPERIMENTAL EYE RESEARCH, vol. 61, no. 3, 1995, pages 273-284, XP002105538
- R.N. LOLLEY ET AL.: "Cyclic GMP Accumulation Causes Degeneration of Photoreceptor Cells: Simulation of an Inherited Disease" SCIENCE, vol. 196, no. 4290, 1977, pages 664-666, XP002105539 cité dans la demande
- J.H. STERN ET AL.: "Control of the light-regulated current in rod receptor by cyclic GMP, calcium, and l-cis-diltiazem" PROC. NATL. ACAD. SCI., vol. 83, no. 4, 1986, pages 1163-1167, XP002105540
- B.S. PAWLYK ET AL.: "Effects of IBMX on the rod ERG of the isolated perfused cat eye: antagonism with light, cacium or l-cic-diltiazem" VISION RES., vol. 31, no. 7-8, 1991, pages 1093-1098, XP002105541

## Description

La présente invention a pour objet l'utilisation d'un composé bloqueur des canaux calciques et/ou de canaux activés par la guanosine monophosphate cyclique 3' 5' (cGMP) dans le cadre du traitement de pathologies de la rétine dues à une dégénérescence des photorécepteurs, chez l'homme ou l'animal, telles que la rétinite pigmentaire ou autres pathologies touchant semblablement les photorécepteurs, notamment la dégénérescence maculaire liée à l'âge.

La rétinite pigmentaire désigne un ensemble de ces maladies dégénératives des photorécepteurs (Berson, 1996) conduisant à la cécité.

De nombreuses mutations affectant différentes protéines des bâtonnets, et susceptibles d'être à l'origine de cette maladie, ont été mises en évidence. Parmi ces mutations, on peut citer celles affectant les gènes de protéines impliquées dans la cascade de la phototransduction, telles que la rhodopsine, la transducine, la phosphodiestérase, l'arrestine, ou de protéines structurales, telle que la périphérine.

La souris *rd* (retinal degeneration) a été étudiée pendant plus de 70 ans comme modèle de la rétinite pigmentaire (Farber et coll., 1994) car le processus de dégénérescence rétinienne est similaire à celui observé dans la rétine pigmentaire, la mort des bâtonnets de la rétine étant suivie par une perte inexpliquée des cônes de la rétine. De plus, la mutation causale a été localisée dans le gène codant pour la sous-unité β de la cGMP-phosphodiestérase (PDE) (Bowes et coll., 1990), comme dans certaines familles affectées par la maladie (Mc Laughlin et coll., 1993).

La PDE est activée durant la cascade de phototransduction par la chaîne α de la transducine, elle-même activée par la rhodopsine stimulée par la lumière. La PDE activée hydrolyse le cGMP, réduisant ainsi la concentration de cGMP et donc le nombre des canaux cGMP-dépendants ouverts, la conséquence finale étant une diminution de la conductance aux cations tels que Na⁺ et Ca²⁺ d'où une réduction du courant de dépolarisation des photorécepteurs dans l'obscurité. Chez la souris *rd*, Farber et Lolley (1974) ont montré qu'une augmentation anormale de la concentration de cGMP précède la dégénérescence des photorécepteurs. La toxicité du cGMP à forte concentration a ensuite été établie sur des photorécepteurs normaux (Lolley et Farber, 1977; Ulshafer et coll., 1980).

Quelques approches thérapeutiques destinées à prévenir la perte de photorécepteurs sont actuellement en cours d'investigation sur les souris *rd*. Il a ainsi été décrit que la thérapie génique *in vivo* permet de retarder la mort des photorécepteurs pendant six semaines après injection sous-rétinienne d'un adénovirus recombinant à réplication défectueuse qui contient l'ADNc codant la PDE murine (Bennett et coll., 1996). La transplantation de photorécepteurs (Gouras et coll., 1994, Silverman et coll., 1989) a été décrite comme permettant de préserver les photorécepteurs des cônes (Mohand-Said et coll., 1997). L'interprétation de cet effet en tant que mécanisme paracrine est en corrélation avec l'augmentation de la survie des photorécepteurs observée en coculture avec des photorécepteurs sains (Mohand-Said et coll., 1998) ou après application *in vivo* ou *in vitro* de facteurs trophiques tels que les facteurs de croissance fibroblastiques ou neuronaux (LaVail et coll., 1998).

Cependant, aucun traitement des maladies de la rétine dues à une dégénérescence des photorécepteurs, n'est actuellement disponible, mise à part la prescription de vitamine A pour la rétinite pigmentaire (Berson, 1996).

La présente invention a précisément pour but de fournir des compositions pharmaceutiques utilisables dans le cadre du traitement de maladies de la rétine dues à une dégénérescence des photorécepteurs chez l'homme ou l'animal.

En effet, la présente invention découle de la mise en évidence par les Inventeurs du fait que les composés bloqueurs des canaux calciques et/ou cGMP-dépendants, tels que le chlorhydrate de diltiazem, permettent non seulement de ralentir la dégénérescence des bâtonnets ***et des cônes*** chez la souris *rd*, mais également de préserver la capacité des cellules rétiniennes à répondre aux stimuli lumineux.

L'invention a pour objet l'utilisation du diltiazem (D-cis-énantiomère), du L-cis-énantiomère, de leurs métabolites et de leurs sels pharmaceutiquement acceptables, pour la préparation d'un médicament destiné au traitement des pathologies liées à la dégénérescence des photorécepteurs de la rétine, notamment au traitement de la rétinite pigmentaire, ou de pathologies touchant semblablement les photorécepteurs, telle que la dégénérescence maculaire liée à l'âge.

L'invention concerne plus particulièrement l'utilisation susmentionnée du diltiazem de formulé 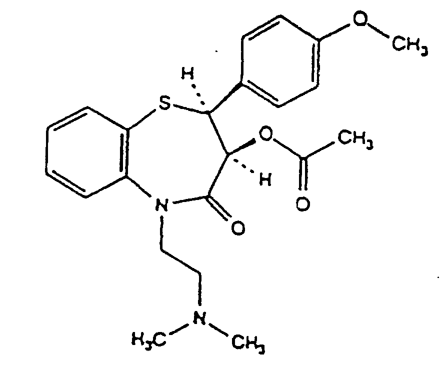 ainsi que ses sels d'addition aux acides pharmaceutiquement acceptables, notamment le malate ou le chlorhydrate de diltiazem.

L'invention a également pour objet l'utilisation susmentionnée du L-cis énantiomère du diltiazem, ou le racémate du diltiazem, ainsi que leurs sels d'addition aux acides pharmaceutiquement acceptables, notamment le chlorhydrate de l'isomère cis(-) de formule suivante :

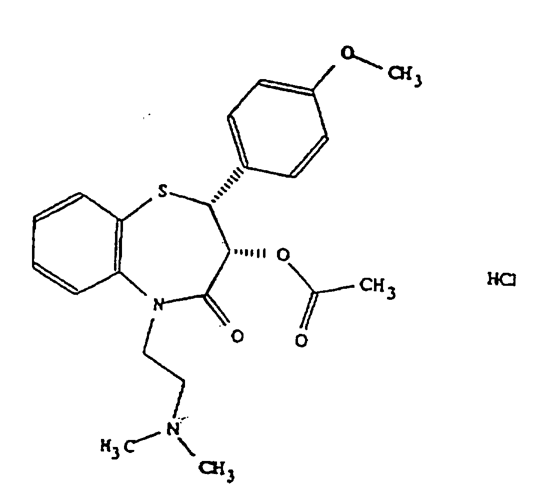

Avantageusement, les composés bloqueurs des canaux calciques et/ou cGMP-dépendants susmentionnés sont utilisés pour la préparation de compositions pharmaceutiques se présentant sous une forme administrable par toute voie, notamment par voie orale, intramusculaire, intraveineuse, intraoculaire, ou sous forme de collyre.

De préférence les compositions pharmaceutiques de l'invention comprennent, sous forme unitaire, environ 0,1 à environ 100 mg d'un composé bloqueur des canaux calciques et/ou cGMP-dépendants, tel que défini ci-dessus, en association avec un véhicule pharmaceutiquement acceptable.

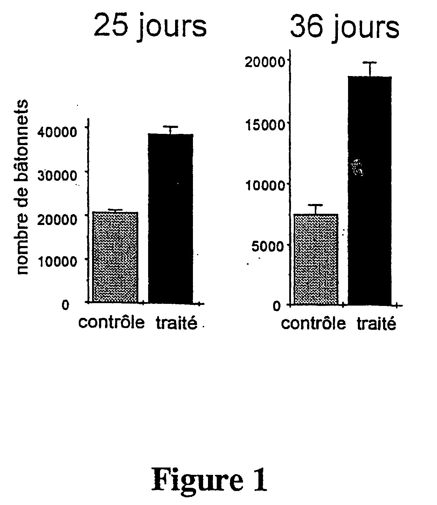

L'invention sera davantage illustrée à l'aide des figures 1 à 3 suivantes.

La figure 1 illustre la survie des bâtonnets au 25° jour et au 36° jour postnatal chez les souris *rd* traitées par le chlorhydrate de diltiazem. Les injections de chlorhydrate de diltiazem ont débutés au 9° jour postnatal qui correspond à la période d'apparition des premiers signes de dégénérescence des bâtonnets. Les doses de chlorhydrate de diltiazem (2,5 mg/ml dans une solution physiologique) ont été augmentées graduellement à partir de 50µl par jour jusqu'à 100 µl deux fois par jour suivant la croissance de l'animal. Puisque les électrorétinogrammes (ERG) ont été mesurés avant le sacrifice des animaux, la dernière injection a été donnée 48 heures avant la mesure physiologique. Après fixation des rétines, les bâtonnets ont été marqués par des anticorps anti-rhodopsine (rho-4D2) (Hicks and Molday, 1986) et leur nombre a été estimé par stéréologie sur les rétines mises à plat en utilisant une procédure d'échantillonnage aléatoire (Mohand-Said et coll., 1998). Seules les rétines droites ont été prises en considération afin d'obtenir des résultats indépendants. Le traitement au chlorhydrate de diltiazem a augmenté de 86 % la survie des bâtonnets au 25° jour, et de 148 % au 36° jour chez les animaux traités par rapport aux souris *rd* de contrôle. Des injections répétées de solution physiologique seule n'ont pas affecté de façon significative la survie des bâtonnets (7416 ± 1291, s.e.m., n = 5) par rapport aux animaux non traités (7648 ± 774, s.e.m., n = 4). Ces observations montrent donc que le traitement des souris *rd* par le chlorhydrate de diltiazem induit une survie des bâtonnets.

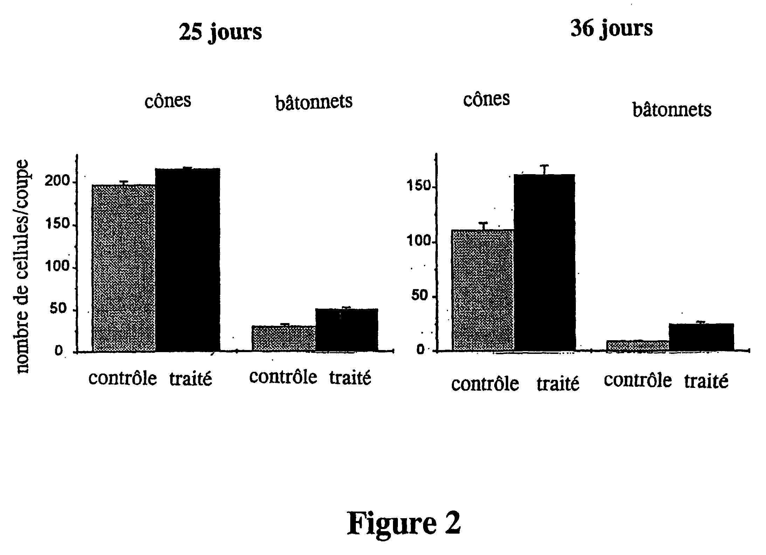

La figure 2 illustre la survie des cônes au 25° jour et au 36° jour postnatal chez les souris rd traitées par le chlorhydrate de diltiazem. Le nombre de cônes a été estimé indirectement après coloration des noyaux avec le DAPI (4',6-diamino-2-phénylindole) sur des coupes de rétines. Pour obtenir ce nombre, le nombre de bâtonnets immunomarqués a été soustrait du nombre de noyaux de photorécepteurs marqués au DAPI. Le traitement au chlorhydrate de diltiazem a augmenté de 109 % la survie des cônes au 25° jour, et de 144 % au 36° jour chez les animaux traités par rapport aux souris rd de contrôle.

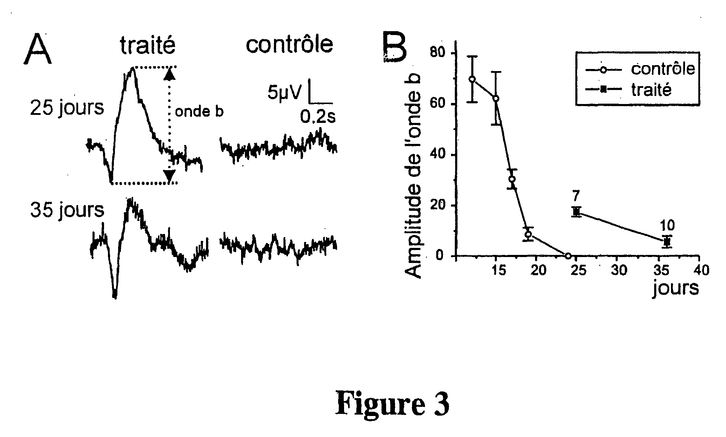

La figure 3 illustre le fait que la survie des photorécepteurs est accompagnée d'améliorations physiologiques chez les souris *rd* traitées par le chlorhydrate de diltiazem. Cette démonstration a été effectuée par mesure des ERG chez les souris rd traitées et les souris *rd* de contrôle. Chez des souris rd non traitées, les amplitudes des ondes a et b des ERG diminuent régulièrement à partir du 12° jour postnatal jusqu'à extinction au 24° jour postnatal. En revanche, tous les animaux traités présentent des signaux ERG AU 25° jour postnatal (n = 7). Au 36° jour postnatal, 4 animaux traités sur 10 présentent des signaux ERG pouvant être mesurés dans les deux yeux. Ces observations montrent que le traitement par le chlorhydrate de diltiazem non seulement permet aux bâtonnets de survivre mais protège aussi les fonctions visuelles de la rétine.

Légende des figures :
- Figure 1 : estimation du nombre de bâtonnets sur des rétines entières de souris *rd* de 25 jours et de 36 jours traitées par le chlorhydrate de diltiazem, par rapport aux souris *rd* de contrôle non traitées.
- Figure 2 : estimation du nombre de cônes et de bâtonnets sur des coupes de rétines de souris rd de 25 jours et de 36 jours traitées par le chlorhydrate de diltiazem, par rapport aux souris rd de contrôle non traitées.
- Figure 3 : effet du chlorhydrate de diltiazem sur l'ERG mesuré chez les souris *rd*. A) représentation des enregistrements d'ERG mesurés chez les souris *rd* traitées et de contrôle au 25° et au 36° jour postnatal. B) effet du chlorhydrate de diltiazem sur l'onde b de l'ERG ; la courbe correspondant à la mesure de l'amplitude de l'onde b en fonction du temps chez les souris *rd* de contrôle est représentée à l'aide de cercles creux, tandis que celle correspondant à cette même mesure chez les souris *rd* traitées par le chlorhydrate de diltiazem est représentée à l'aide de carrés noirs.

### REFERENCES BIBLIOGRAPHIQUES

Bennett J., Tanabe T., Sun D., Zeng Y., Kjeldbye H., Gouras P., Maguire A.M. ; "Photoreceptor cell rescue in retinal degeneration (*rd*) mice by in vitro gene therapy", Nat. Med. (1996) 2 : 649-654.

Berson E.L. ; "Retinitis pigmentosa : Unfolding its mystery", Proc. Natl. Acad. Sci. (1996) 93 : 4526-4528.

Bowes C., Li T., Danciger M., Baxter L.C., Applebury M.L., Farber D. ; "Retinal degeneration in the rd mouse is caused by a defect in the β-subunit of rod cGMP-phosphodiesterase" ; Nature (1990) 347 : 677-680.

Farber D.B. and Lolley RN. ; Cyclic guanoside monophosphate : Elevation in degenerating photoreceptor cells of the C3H mouse retina. Science (1974) 186 : 449-451.

Farber D.B., Flannery J.G., Bowes-Rickman C. ; "The rd mouse story : seventy years of research on an animal model of inherited retinal degeneration", Prog. Ret. Eye Res. (1994) 31-62.

Gouras P., Du J., Kjeldbye H., Yamamoto S., Zack D.J. ; "Long-term photoreceptor transplants in dystrophic and normal mouse retina", Invest. Ophthalmol. Vis. Sci. (1994) 35 : 3145-53.

Hicks D. and Molday R.S. ; Differential immunogold-dextran labeling of bovine and frog rod and cones cells using monoclonal antibodies against bovine rhodopsin. Experimental Eye Research (1986) 42: 55-71.

Lavail M. M., Yasumura D., Matthes M. T., Lau-Villacorta C., Unoki K., Sung C. H., Steinberg R. H. Protection of mouse photoreceptors by survival factors in retinal degenerations. Invest. Ophthal. Vis. Sci. (1998) 39 : 592-602.

Lolley R.N., Farber D.B., Raybom M.E. and Hollyfield J.G. ; Cyclic GMP accumulation causes degeneration of photoreceptor cells : simulation of an inherited disease. Science (1977) 196 : 664-666.

McLaughlin M.E., Sandberg M.A., Berson E.L., Drya T.P. ; "Recessive mutations in the gene encoding the b-subunit of rod phosphodiesterase in patients with retinitis pigmentosa", Nature genetics (1993) 4 : 130-133.

Mohand-Said S., Hicks D., Simonutti M., Tran-Minh D., Deudon-Combe A., Dreyfus H., Silvermann M.S., Mosinger Ogilvie J., Tenkova T., Sahel J. ; "Photoreceptor transplants increase host cone survival in the retinal degeneration (*rd*) mouse", Ophth. Res. (1997) 29 : 290-297.

Mohand-Said S., Deudon-Combe A., Hicks D., Simonutti M., Forster V., Fintz A.C., Léveillard T., Dreyfus H. and Sahel J. ; Normal retina releases a diffusible factor stimulating cone survival in the retinal degeneration mouse. Proceeding of the National Academy of Sciences (USA) (1998) 95 : 8357-8362.

Ulshafer R.J., Garcia C.A. and Hollyfield J.G. ; Sensitivity of photoreceptors to elevated levels of cGMP in the human retina. Investigative Ophthalmology and Visual Science (1980) vol. 19, n° 10: 1236-1241.

## Revendications

1. Utilisation du diltiazem (D-cis-énantiomère), du L-cis-énantiomère, et de leurs sels pharmaceutiquement acceptables, pour la préparation d'un médicament destiné au traitement des pathologies liées à la dégénérescence des photorécepteurs de la rétine.

2. Utilisation du diltiazem (D-cis-énantiomère), du L-cis-énantiomère, et de leurs sels pharmaceutiquement acceptables, pour la préparation d'un médicament destiné au traitement de la rétinite pigmentaire, ou de pathologies touchant semblablement les photorécepteurs, telle que la dégénérescence maculaire liée à l'âge.

3. Utilisation selon la revendication 1 ou 2, du diltiazem de formule 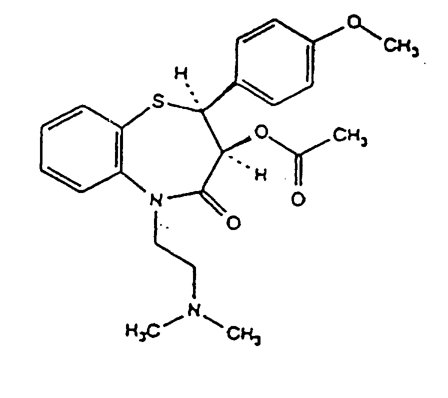 ainsi que de ses sels d'addition aux acides pharmaceutiquement acceptables, notamment le malate ou le chlorhydrate de diltiazem.

4. Utilisation selon la revendication 1 ou 2, du L-cis énantiomère, ou du racémate, ainsi que leurs sels d'addition aux acides pharmaceutiquement acceptables.

## Claims

1. Use of diltiazem (D-cis-enantiomer), L-cis-enantiomer, and their pharmaceutically acceptable salts, for preparing a medicament for the treatment of pathologies related to degeneration of the retinal photoreceptors.

2. Use of diltiazem (D-cis-enantiomer), L-cis-enantiomer, and their pharmaceutically acceptable salts, for preparing a medicament intended to treat retinitis pigmentosa, or pathologies substantially related to the photoreceptors, such as age-related macular degeneration.

3. Use according to claim 1 or 2, of diltiazem of the formula 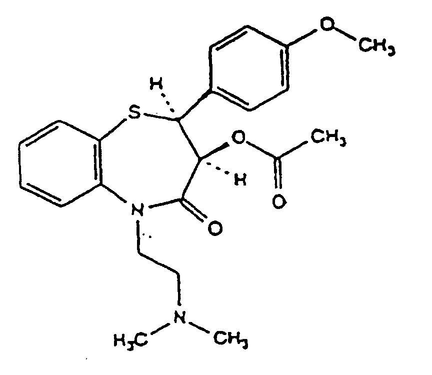 as well as pharmaceutically acceptable acid addition salts, especially the malate or hydrochloride of diltiazem.

4. Use according to claim 1 or 2, of the L-cis enantiomer, or of the racemate, as well as their pharmaceutically acceptable acid addition salts.

## Patentansprüche

1. Verwendung von Diltiazem (D-cis-énantiomér), von L-cis-énantiomér und ihren pharmazeutisch annehmbaren Salzen für die Herstellung eines Medikaments für die Behandlung von Pathologien, die mit der Degeneration von Photorezeptoren der Netzhaut verbunden sind.

2. Verwendung von Diltiazem (D-cis-énantiomér), von L-cis-enantiomer und ihren pharmazeutisch annehmbaren Salzen für die Herstellung eines Medikaments für die Behandlung von Retinits pigmentosa oder von Pathologien, die die Photorezeptoren in ähnlicher Weise angreifen wie die altersbedingte Makulardegeneration.

3. Verwendung nach Anspruch 1 oder 2 von Diltiazem der Formel 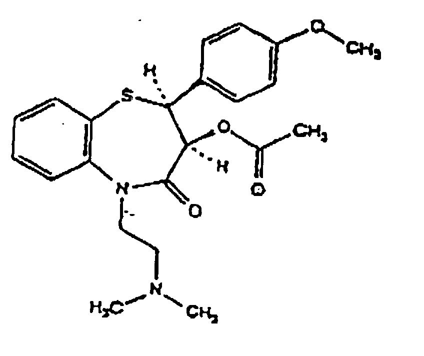 sowie seiner Additionssalze pharmazeutisch annehmbarer Säuren, insbesondere das Malat oder das Chlorhydrat von Diltiazem.

4. Verwendung nach Anspruch 1 oder 2 von L-cis-énantiomér oder des Razemats sowie ihrer Additionssalze pharmazeutisch annehmbarer Säuren.
